# FASCICULE DE BREVET EUROPEEN

(11) **EP 0 595 370 B1**
(45) Date de publication et mention de la délivrance du brevet: **25.02.1998**
(21) Numéro de dépôt: 93119434.4
(22) Date de dépôt: 22.11.1991
(51) Int. Cl.: C07D 305/14, C07D 263/04

(54) **Procédé de préparation de dérivés du taxane et intermédiaires**
Verfahren zur Herstellung von Taxanderivaten und deren Zwischenprodukte
Process for the preparation of taxane derivatives and intermediates therefor

(30) Priorité: 23.11.1990 FR 9014635; 25.07.1991 FR 9109423
(43) Date de publication de la demande: 04.05.1994
(62) Demande divisionnaire de: 92900928.0
(73) Titulaire: RHONE-POULENC RORER S.A., 92160 Antony (FR)
(72) Inventeur: Bourzat, Jean-Dominique, F-94300 Vincennes (FR); Commercon, Alain, F-94400 Vitry Sur Seine (FR); Paris, Jean-Marc, F-77360 Vaires Sur Marne (FR)
(74) Mandataire: Pilard, Jacques

(56) Documents cités:
- EP-A- 0 253 739
- EP-A- 0 336 841
- JOURNAL OF NATURAL PRODUCTS, vol. 51, no. 2 , 1988 Columbus, Ohio, US, pages 298 - 306 N.F. MAGRI, ET AL.: 'modified taxols. 4. Synthesis and biological activity of taxols modified in the side-chain'

## Description

La présente invention concerne un procédé de préparation de dérivés du taxane de formule générale : 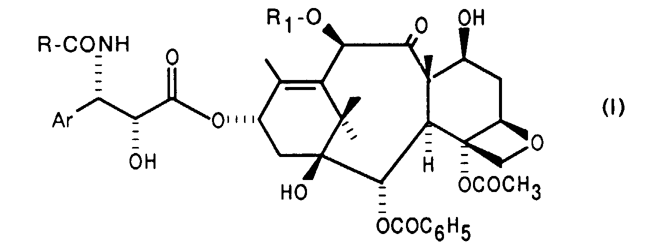 dans laquelle R représente un radical t.butoxy ou phényle, R₁ représente un atome d'hydrogène ou un radical acétyle et Ar représente un radical phényle.

Dans EP-A-0 253 739 est décrite la préparation du taxol à partir du Taxotère (t.butoxycarbonylamino-3 phényl-3 hydroxy-2 propionate-(2R,3S) d'acétoxy-4 benzoyloxy-2 époxy-5β,20 dihydroxy-7β,10β oxo-9 taxène-11 yle-13α) qui peut lui-même être obtenu selon le procédé décrit dans EP-A-0 336 841 par condensation de l'acide t.butoxycarbonylamino-3 phényl-3 hydroxy (protégé)-2 sur la désacétyl-10 baccatine III convenablement protégée. Le taxol et le Taxotère présentent des propriétés antitumorales et antileucémiques. D'après Chem. Abstr. 114 94569 q (1991), les métabolites du taxol qui sont des dérivés hydroxylés sur le noyau phényle en -3' ou sur le noyau phényle du benzoyle en -2 sont nettement moins actifs que le taxol.

Selon l'invention, les dérivés du taxane de formule générale (I) peuvent être obtenus de la manière suivante :
1) le dérivé de l'oxazolidine de formule générale : 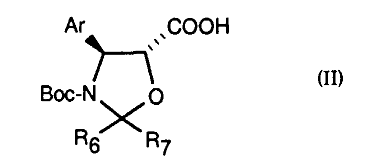 dans laquelle Ar est défini comme précédemment, Boc représente le radical t.butoxycarbonyle et R₆ et R₇, identiques ou différents, représentent un radical alkyle contenant 1 à 4 atomes de carbone éventuellement substitué par un ou plusieurs radicaux aryles (phényle), ou aryle (phényle), ou bien R₆ et R₇ forment ensemble avec l'atome de carbone auquel ils sont liés un cycle ayant de 4 à 7 chaînons, est condensé avec un dérivé du taxane de formule générale : 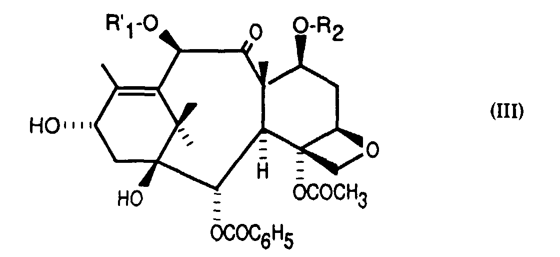 dans laquelle R'₁ représente un radical acétyle ou un groupement protecteur de la fonction hydroxy tel que le radical trichloro-2,2,2 éthoxycarbonyle et R₂ représente un groupement protecteur de la fonction hydroxy tel que le radical trichloro-2,2,2 éthoxycarbonyle ou un radical trialcoylsilyle dont chaque partie alkyle contient 1 à 4 atomes de carbone pour donner le produit de formule générale : 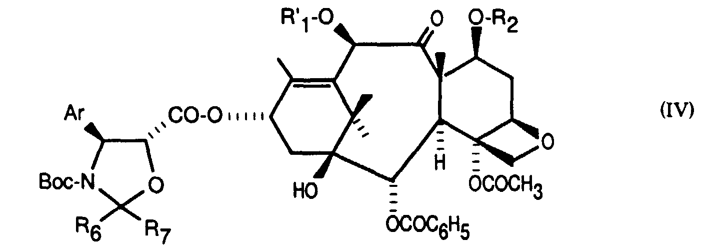 dans laquelle Ar, R'₁, R₂, R₆ et R₇ sont définis comme précédemment.

Généralement, l'estérification est effectuée en présence d'un agent de condensation tel qu'un carbodiimide comme le dicyclohexylcarbodiimide ou un carbonate réactif comme le dipyridyl-2 carbonate et d'un agent d'activation tel qu'une dialkylaminopyridine comme la diméthylamino-4 pyridine en opérant dans un solvant organique tel que le benzène, le toluène, les xylènes, l'éthylbenzène, l'isopropylbenzène ou le chlorobenzène à une température comprise entre 60 et 90°C.

Il est particulièrement avantageux d'utiliser un excès molaire d'acide de formule (II) par rapport au dérivé du taxane de formule (III), l'agent de condensation étant utilisé en quantité stoechiométrique par rapport à l'acide de formule (II) et l'agent d'activation étant utilisé en quantité stoechiométrique par rapport au dérivé du taxane de formule (III).

Le produit de formule générale (IV) dans laquelle R'₁ est défini comme précédemment et R₂ représente un radical trichloro-2,2,2 éthoxycarbonyle peut être obtenu par estérification d'un dérivé du taxane de formule générale (III) dans laquelle R'₁ est défini comme précédemment et R₂ représente un radical trialkylsilyle, suivi du remplacement du radical trialkylsilyle par un radical trichloro-2,2,2 éthoxycarbonyle en passant intermédiairement par le produit de formule générale : 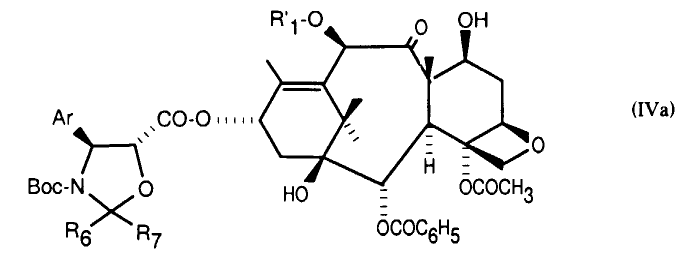 dans laquelle Ar, R'₁, R₆ et R₇ sont définis comme précédemment.

L'estérification peut être effectuée dans les conditions décrites précédemment.

Le produit de formule générale (IVa) peut être obtenu en traitant l'ester de formule générale (IV) dans laquelle R₂ représente un radical trialkylsilyle dont chaque partie alkyle contient 1 à 4 atomes de carbone par l'acide chlorhydrique gazeux dans un alcool tel que l'éthanol.

Le produit de formule générale (IV) dans laquelle R₂ représente un radical trichloro-2,2,2 éthoxycarbonyle peut être obtenu en traitant le produit de formule générale (IVa) par le chloroformiate de trichloro-2,2,2 éthyle en opérant dans un solvant organique basique tel que la pyridine.
2) le produit de formule générale (IV) dans laquelle R₂ représente un radical trichloro-2,2,2 éthoxycarbonyle est traité par un acide minéral ou organique éventuellement dans un alcool dans des conditions qui sont sans effet sur les groupements protecteurs R'₁ et R₂ de façon à obtenir le produit de formule générale : 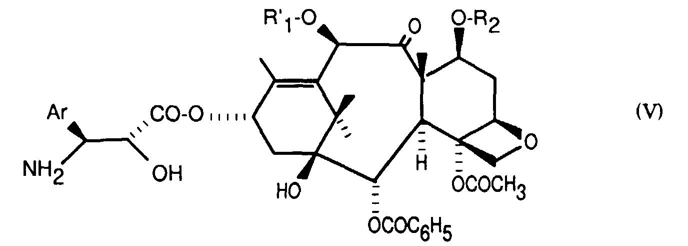 dans laquelle Ar et R'₁ sont définis comme précédemment et R₂ représente un radical trichloro-2,2,2 éthoxycarbonyle.

Généralement, on utilise l'acide formique éventuellement dans un alcool tel que l'éthanol ou l'acide chlorhydrique gazeux dans un alcool tel que l'éthanol,
3) le produit de formule générale (V) est traité par un composé qui permet d'introduire, sur la fonction amino, un radical t.butoxycarbonyle ou benzoyle pour obtenir le produit de formule : 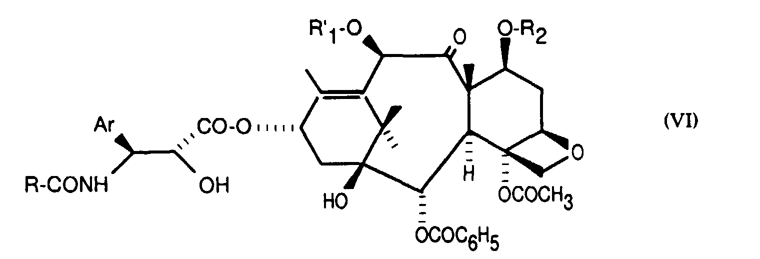 dans laquelle Ar, R et R'₁ sont définis comme précédemment et R₂ représente un radical trichloro-2,2,2 éthoxycarbonyle.

Généralement on fait réagir le dicarbonate de di-tert.butyle ou le chlorure de benzoyle sur le produit de formule générale (VI) en opérant dans un solvant organique tel que le chlorure de méthylène en présence d'une base minérale telle que le bicarbonate de sodium ou d'une base organique telle qu'une amine tertiaire comme la triéthylamine,
4) le produit de formule générale (VI) est transformé en produit de formule générale (I) par remplacement des groupements trichloro-2,2,2 éthoxycarbonyle représentés par R'₁ et R₂ par des atomes d'hydrogène sans toucher au reste de la molécule.

Généralement le produit de formule générale (VI) est traité par le zinc en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone en présence de zinc.

Le produit de formule générale (III) peut être préparé dans les conditions décrites dans le brevet européen EP 0 336 841.

L'acide de formule générale (II) peut être obtenu par saponification en milieu basique de l'ester de formule générale : 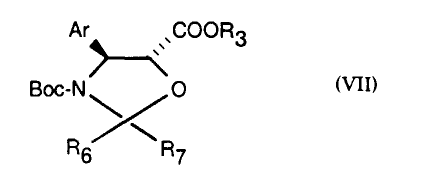 dans laquelle Ar, R₆ et R₇ sont définis comme précédemment et R₃ représente un radical alcoyle contenant 1 à 4 atomes de carbone éventuellement substitué par un radical phényle.

Généralement, la saponification est effectuée au moyen d'une base minérale telle qu'un hydroxyde de métal alcalin (lithium, sodium, potassium), un carbonate ou bicarbonate de métal alcalin (bicarbonate de sodium, carbonate ou bicarbonate de potassium) en milieu hydro-alcoolique tel qu'un mélange méthanol-eau, à une température comprise entre 10 et 40°C, de préférence voisine de 20°C.

L'ester de formule générale (VII) peut être obtenu par action d'un méthoxy alcène éventuellement substitué par un ou plusieurs radicaux aryles (méthoxy-2 propène), d'un gem-diméthoxy alcane éventuellement substitué par un ou plusieurs radicaux aryles (diméthoxy-2,2 propane) ou un gem-diméthoxycycloalcane contenant 4 à 7 atomes de carbone sur un dérivé de la phénylisosérine de formule générale: 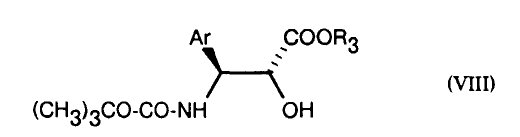 dans laquelle Ar et R₃ sont définis comme précédemment sous forme racémique ou, de préférence, sous forme 2R,3S.

Généralement, la réaction du méthoxy alcène ou du gem-diméthoxy alcane ou du gem-diméthoxycycloalcane avec le produit de formule générale (VIII) est effectuée en opérant dans un solvant organique inerte en présence d'un acide fort tel que l'acide p.toluènesulfonique à une température comprise entre 0°C et la température d'ébullition du mélange réactionnel. Les solvants qui conviennent particulièrement bien sont choisis parmi les hydrocarbures aromatiques (benzène, toluène, xylène).

Le produit de formule générale (VIII) peut être obtenu par acylation d'un dérivé de la β-phénylisosérine de formule générale : 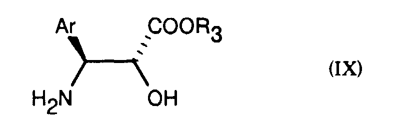 dans laquelle Ar et R₃ sont définis comme précédemment.

La réaction est généralement mise en oeuvre en faisant réagir le dicarbonate de di-tert.butyle en opérant dans un solvant organique inerte tel qu'un ester comme l'acétate de méthyle ou d'éthyle à une température comprise entre 0 et 40°C, de préférence voisine de 20°C.

Le dérivé de la β-phénylisosérine de formule générale (IX) peut être obtenu par réduction d'un hydroxy-azoture de formule générale : 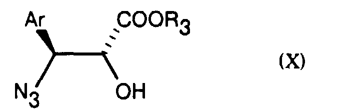 dans laquelle Ar et R₃ sont définis comme précédemment.

Généralement la réduction est effectuée au moyen d'hydrogène en présence d'un catalyseur tel que le palladium sur charbon en opérant dans un solvant organique inerte tel que l'acétate d'éthyle. On opère de préférence à une température comprise entre 0 et 50°C. Il est avantageux d'effectuer l'hydrogénation sous une pression comprise entre 1 et 5 bars.

Le produit de formule générale (X) peut être obtenu par action d'un azoture de métal alcalin tel que l'azoture de sodium sur un ester de l'acide β-phénylglycidique de formule générale : 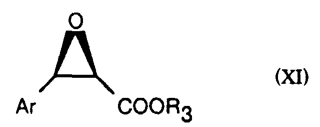 dans laquelle Ar et R₃ sont définis comme précédemment.

Généralement, on opère dans un mélange hydroorganique tel qu'un mélange eau-tétrahydrofuranne à la température de reflux du mélange réactionnel.

L'ester de formule générale (XI) peut être obtenu par déhydrohalogénation d'un produit de formule générale : 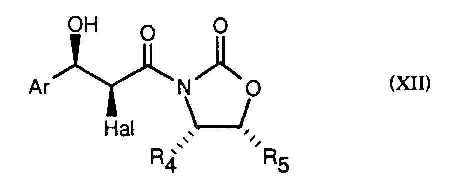 dans laquelle Ar est défini comme précédemment, Hal représente un atome d'halogène, de préférence un atome de brome et R₄ et R₅, identiques ou différents, représentent un radical alcoyle contenant 1 à 4 atomes de carbone ou phényle.

Généralement, on opère en présence d'un excès d'un alcoolate de métal alcalin, éventuellement préparé in situ, dans un solvant organique inerte tel que le tétrahydrofuranne à une température comprise entre -80°C et +25°C.

Le produit de formule générale (XII) peut être obtenu par action d'un aldéhyde de formule générale :

Ar-CHO (XIII)

dans laquelle Ar est défini comme précédemment sur un halogénure de formule générale : 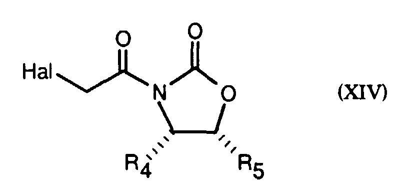 dans laquelle Hal, R₄ et R₅ sont définis comme précédemment, préalablement anionisé.

Généralement, on opère dans un solvant organique inerte choisi parmi les éthers (éther éthylique) et les hydrocarbures aliphatiques halogénés (chlorure de méthylène) à une température comprise entre -80°C et 25°C, en présence d'une amine tertiaire (triéthylamine) et d'un agent d'énolisation (triflate de di-n-butylbore).

Le produit de formule générale (XIV) peut être obtenu par action d'un halogénure d'un acide halogénoacétique, de préférence, le bromure de l'acide bromoacétique sur l'oxazolidinone correspondante.

Les spectres de résonance magnétique nucléaire du proton sont effectués dans la chloroforme deutéré. Selon la nature de la chaîne latérale, la numérotation des atomes est la suivante :

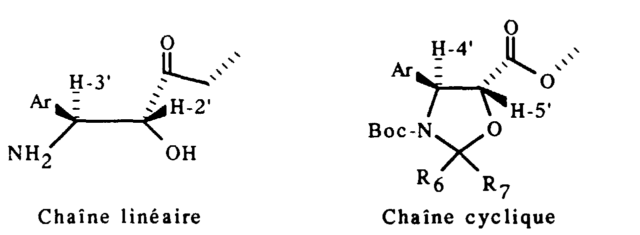

Les abréviations utilisées ont les significations suivantes :
- s =: singulet
- d =: doublet
- dd =: doublet de doublet
- t =: triplet
- q =: quadruplet
- m =: massif
Les exemples suivants illustrent la présente invention.

### EXEMPLE 1

A une solution de 5,52 g d'acide tert-butoxycarbonyl-3 diméthyl-2,2 phényl-4 oxazolidinecarboxylique-5-(4S,5R) dans 350 cm3 de toluène on ajoute 3,55 g de N,N'-dicyclohexylcarbodiimide. La solution est agitée 10 minutes à une température voisine de 20°C, puis on ajoute 3 g de diacétoxy-4,10β benzoyloxy-2α époxy-5β,20 dihydroxy-1,13α oxo-9 triéthylsilyloxy-7β taxène-11 [préparé selon la méthode décrite par J-N. Denis et al., J. Am. Chem. Soc., 110, 5917 (1988)] et 0,52 g de diméthylamino-4 pyridine. Le milieu réactionnel est ensuite chauffé pendant 2 heures à une température voisine de 80°C, après avoir refroidi le mélange réactionnel à une température voisine de 20°C on ajoute 250 cm3 d'une solution aqueuse saturée d'hydrogènocarbonate de sodium. La phase aqueuse est décantée puis extraite avec 2 fois 200 cm3 de dichlorométhane. Les phases organiques réunies sont séchées sur du sulfate de magnésium, filtrées puis concentrées à sec sous pression réduite (2,7 kPa) à une température voisine de 30°C. On obtient 10,8 g d'une huile orange qui est purifiée par chromatographie flash (éluant: dichlorométhane-méthanol [99,5-05 en volumes]). Après concen-tration à sec sous pression réduite (2,7 kPa) à une température voisine de 40°C des fractions 60 à 73 on obtient 3,7 g de tert-butoxycarbonyl-3 diméthyl-2,2 phényl-4 oxazolidine-carboxylate-5-(4S,5R) de diacétoxy-4,10β benzoyloxy-2α époxy-513,20 hydroxy-1 oxo-9 triéthylsilyloxy-7β taxène-11 yle-13α sous forme d'une meringue jaune dont les caractéristiques sont les suivantes :
- pouvoir rotatoire : [α]²⁰_{D} = -45,3° (c = 0,5 ; méthanol)
- spectre de RMN (400 MHz ; déplacements en ppm)

| | |
|---|---|
| 7,35 ppm | m, 5H, C₆H₅ (chaîne) |
| 6,5 ppm s, 1H, 11-10 | |
| 6,25 ppm | t, 1H, H-13 |
| 5,15 ppm | m, 1H, CHN (H-4') |
| 4,5 ppm m, 2H, CHO (H-5')+ H-7 | |
| 4,15 et 4,25 ppm | 2d, 2H, H-20 |
| 1,25 ppm | s, 15H, C(CH₃)₃ + 2 CH₃ du cycle taxène |
| 0,98 ppm | t, 9H, CH₃CSi |
| 0,6 ppm q, 6H, CH₂Si | |

3 g de tert-butoxycarbonyl-3 diméthyl-2,2 phényl-4 oxazolidinecarboxylate-5-(4S,5R) de diacétoxy-4,10β benzoyloxy-2α époxy-5β,20 hydroxy-1 oxo-9 triéthylsilyloxy-7β taxène-11 yle-13α sont ajoutés à 60 cm3 d'une solution éthanolique 0,1N d'acide chlorhydrique gazeux maintenue à 0°C. Le milieu réactionnel est agité 48 heures à 0°C, on ajoute ensuite 250 cm3 de dichlorométhane, puis un mélange de 30 cm3 d'une solution aqueuse saturée d'hydrogènocarbonate de sodium et de 30 cm3 d'eau. La phase organique est décantée, séchée sur du sulfate de magnésium, filtrée puis concentrée à sec sous pression réduite (2,7 kPa) à une température voisine de 30°C. On obtient 2,8 g d'une meringue blanche qui est purifiée par chromatographie flash (éluant : dichlorométhane-méthanol [99-1 en volumes]). Après concentration à sec sous pression réduite (2,7 kPa) à une température voisine de 40°C des fractions 15 à 30, on obtient 2,33 g de tert-butoxycarbonyl-3 diméthyl-2,2 phényl-4 oxazolidinecarboxylate-5-(4S,5R) de diacétoxy-4,10β benzoyloxy-2α époxy-5β,20 dihydroxy-1,7β oxo-9 taxène-11 yle-13α sous forme d'une meringue jaune dont les caractéristiques sont les suivantes :
- pouvoir rotatoire : [α]²⁰_{D} = -65,1° (c = 1 ; méthanol)
- spectre de RMN (250 MHz ; déplacements en ppm)

| | |
|---|---|
| 7,35 ppm | m, 5H, C₆H₅ (chaîne) |
| 6,3 ppm m, 2H, H-10 et H-13 | |
| 5,1 ppm m, 1H, CHN (H-4') | |
| 4,5 ppm d, 1H, CHO (H-5') | |
| 4,45 ppm | m, 1H, H-7 |
| 4,1 et 4,3 ppm | 2d, 2H, H-20 |
| 1,05 à 1,15 ppm | m, 9H, C(CH₃)₃ |

A une solution de 2,3 g de tert-butoxycarbonyl-3 diméthyl-2,2 phényl-4 oxazolidinecarboxylate-5- (4S,5R) de diacétoxy-4,10β benzoyloxy-2α époxy-5β,20 dihydroxy-1,7β oxo-9 taxène-11 yle-13α dans 35 cm3 de pyridine, on ajoute en 10 minutes goutte à goutte 0,4 cm3 de chloroformiate de trichloro-2,2,2 éthyle. Le milieu réactionnel est chauffé à 80°C pendant 1 heure, puis on ajoute 0,36 cm3 de chloroformiate de trichloro-2,2,2 éthyle. Le milieu réactionnel est maintenu 4 heures à 80°C, on additionne alors 0,36 cm3 de chloroformiate de trichloro-2,2,2 éthyle et on maintient la température à 80°C pendant 30 minutes. Après avoir refroidi le milieu réactionnel à une température voisine de 20°C, on ajoute 175 cm3 d'eau, 175 cm3 d'une solution aqueuse saturée d'hydrogénocarbonate de sodium et 150 cm3 de dichlorométhane. La phase aqueuse est décantée, puis extraite avec 2 fois 25 cm3 de dichlorométhane. Les phases organiques réunies sont séchées sur du sulfate de magnésium, filtrées puis concentrées à sec sous pression réduite (0,13 kPa) à une température voisine de 30°C. On obtient 2,8 g d'une meringue blanche qui est purifiée par chromatographie flash (éluant : dichlorométhane-méthanol [99-1 en volumes]). Après concentration à sec sous pression réduite (2,7 kPa) à une température voisine de 40°C des fractions 28 à 42 on obtient 2,57 g de tert-butoxycarbonyl-3 diméthyl-2,2 phényl-4 oxazolidinecarboxylate-5-(4S,5R) de diacétoxy-4,10β benzoyloxy-2α époxy-5β,20 hydroxy-1 oxo-9 (trichloro-2,2,2 éthoxy)carbonyloxy-7β taxène-11 yle-13α sous forme d'une meringue blanche dont les caractéristiques sont les suivantes :
- pouvoir rotatoire : [α]²⁰_{D} = -55° (c = 0,5 ; méthanol)
- spectre de RMN (400 MHz ; déplacements en ppm)

| | |
|---|---|
| 7,35 ppm | m, 5H, C₆H₅ (chaîne) |
| 6,4 ppm s, 1H, H-10 | |
| 6,25 ppm | t, 1H, H-13 |
| 5,6 ppm dd, 1H, H-7 | |
| 5,1 ppm m, 1H, CHN (H-4') | |
| 5,05 et 4,65 ppm | 2d, 2H, CH₂CCl₃ |
| 4,5 ppm d, 1H, CHO (H-5') | |
| 4,1 et 4,3 ppm | 2d, 2H, H-20 |
| 1,05 à 1,15 ppm | m, 9H, C(CH₃) ₃ |

Une solution de 3,2 g de tert-butoxycarbonyl-3 diméthyl-2,2 phényl-4 oxazolidinecarboxylate-5-(4S,5R) de diacétoxy-4,10β benzoyloxy-2α époxy-5β,20 hydroxy-1 oxo-9 (trichloro-2,2,2 éthoxy) carbonyloxy-7β taxène-11 yle-13α dans 32 cm3 d'acide formique est agitée 4,5 heures à une température voisine de 20°C. Le milieu réactionnel est concentré à sec sous pression réduite (0,13 kPa) à une température voisine de 30°C. Le résidu obtenu est mis en solution dans 150 cm3 de dichlorométhane, cette solution est lavée avec 50 cm3 d'une solution aqueuse saturée d'hydrogènocarbonate de sodium, séchée sur du sulfate de magnésium, filtrée et concentrée à sec sous pression réduite (0,13 kPa) à une température voisine de 30°C. On obtient 2,74 g d'une meringue crème qui est purifiée par chromatographie flash (éluant : dichlorométhane-méthanol [99-1 en volumes]). Après concentration à sec sous pression réduite (2,7 kPa) à une température voisine de 40°C des fractions 13 à 25 on obtient 2,17 g d'amino-3 hydroxy-2 phényl-3 propionate-(2R,3S) de diacétoxy-4,10β benzoyloxy-2α époxy-5β,20 hydroxy-1 oxo-9 (trichloro-2,2,2 éthoxy) carbonyloxy-7β taxène-11 yle-13α sous forme d'une meringue blanche dont les caractéristiques sont les suivantes :
- pouvoir rotatoire : [α]²⁰_{D} = -60,2° (c = 0,5 ; méthanol)
- spectre de RMN (400 MHz ; déplacements en ppm)

| | |
|---|---|
| 7,4 à 7,25 ppm | m, 5H, C₆H₅ (chaîne) |
| 6,4 ppm s, 1H, H-10 | |
| 6,15 ppm | t, 1H, H-13 |
| 5,55 ppm | dd, 1H, H-7 |
| 5,05 et 4,65 ppm | 2d, 2H, CH₂CCl₃ |
| 4,35 ppm | m, 2H, H-2' et H-3' |
| 4,15 et 4,3 ppm | 2d, 2H, H-20 |

L'amino-3 hydroxy-2 phényl-3 propionate-(2R,3S) de diacétoxy-4,10β benzoyloxy-2α époxy-5β,20 hydroxy-1 oxo-9 (trichloro-2,2,2 éthoxy) carbonyloxy-7β taxène-11 yle-13α peut également être préparé de la manière suivante :

Une solution de 0,1 g de tert-butoxycarbonyl-3 diméthyl-2,2 phényl-4 oxazolidinecarboxylate-5-(4S,5R) de diacétoxy-4,10β benzoyloxy-2α époxy-5β,20 hydroxy-1 oxo-9 (trichloro-2,2,2 éthoxy) carbonyloxy-7β taxène-11 yle-13α dans un mélange de 1,4 cm3 d'une solution éthanolique 3,4N d'acide chlorhydrique gazeux et de 0,6 cm3 de dichlorométhane est agitée 7 heures à une température voisine de 20°C. On ajoute alors au milieu réactionnel 2 cm3 de dichlorométhane, 1 cm3 d'eau et 1 cm3 d'une solution aqueuse saturée d'hydrogénocarbonate de sodium. La phase organique est décantée, séchée sur du sulfate de magnésium, filtrée et concentrée à sec sous pression réduite (0,13 kPa) à une température voisine de 30°C. On obtient 0,1 g d'une meringue jaune qui est purifiée par chromatographie flash (éluant : dichlorométhane-méthanol [98-2 en volumes]). Après concentration à sec sous pression réduite (2,7 kPa) à une température voisine de 40°C des fractions 14 à 20 on obtient 0,023 g d'amino-3 hydroxy-2 phényl-3 propionate-(2R,3S) de diacétoxy-4,10β benzoyloxy-2α époxy-5β,20 hydroxy-1 oxo-9 (trichloro-2,2,2 éthoxy) carbonyloxy-7β taxène-11 yle-13α sous forme d'une meringue blanche.

A une solution agitée de 2,1 g d'amino-3 hydroxy-2 phényl-3 propionate-(2R,3S) de diacétoxy-4,10β benzoyloxy-2α époxy-5β,20 hydroxy-1 oxo-9 (trichloro-2,2,2 éthoxy) carbonyloxy-7β taxène-11 yle-13α dans 52,5 cm3 d'acétate d'éthyle, on ajoute un mélange de 140 cm3 d'une solution aqueuse saturée d'hydrogénocarbonate de sodium et de 140 cm3 d'eau. On additionne ensuite en 2 minutes 0,277 cm3 de chlorure de benzoyle. Le milieu réactionnel est agité 15 minutes à une température voisine de 24°C, la phase aqueuse est décantée puis extraite avec 3 fois 50 cm3 de dichlorométhane. Les phases organiques réunies sont séchées sur du sulfate de magnésium, filtrées puis concentrées à sec sous pression réduite (2,7 kPa) à une température voisine de 30°C. On obtient 2,3 g d'une meringue crème qui est purifiée par chromatographie flash (éluant : dichlorométhane- méthanol [99-1 en volumes]). Après concentration à sec sous pression réduite (2,7kPa) à une température voisine de 40°C des fractions 31 à 38 on obtient 2,03 g de benzoylamino-3 hydroxy-2 phényl-3 propionate-(2R,3S) de diacétoxy-4,10β benzoyloxy-2α époxy-5β,20 hydroxy-1 oxo-9 (trichloro-2,2,2 éthoxy) carbonyloxy-7β taxène-11 yle-13α sous forme d'une meringue blanche dont les caractéristiques sont les suivantes :
- pouvoir rotatoire : [α]²⁰_{D} = -41,9° (c = 0,5 ; méthanol)
- spectre de RMN (250 MHz ; déplacements en ppm)

| | |
|---|---|
| 8,15ppm | d,2H ] |
| 7,8 ppm d, 2H | ] 2 x C₆H₅CO + C₆H₅ (chaîne) |
| 7,6 ppm t,1H | ] |
| 7,6 à 7,3 ppm | m, 10H ] |
| 7,05 ppm | d, 1H, NH |
| 6,35 ppm | s, 1H, H-10 |
| 6,2 ppm t, 1H, H-13 | |
| 5,8 ppm dd, 1H, H-3' | |
| 5,55 ppm | dd, 1H, H-7 |
| 5,05 et 4,62 ppm | 2d, 2H, CH₂CCl₃ |
| 4,8 ppm d, 1H, H-2' | |
| 4,2 et 4,35 ppm | 2d, 2H, H-20 |

Le benzoylamino-3 hydroxy-2 phényl-3 propionate-(2R,3S) de diacétoxy-4,10β benzoyloxy-2α époxy-5β,20 hydroxy-1 oxo-9 (trichloro-2,2,2 éthoxy) carbonyloxy-7β taxène-11 yle-13α peut être transformé en taxol par remplacement du groupement trichloro-2,2,2 éthoxycarbonyle par un atome d'hydrogène au moyen de zinc en présence d'acide acétique selon la méthode décrite dans le brevet européen EP 0 253 738.

L'acide tert-butoxycarbonyl-3 diméthyl-2,2 phényl-4 oxazolidinecarboxylique-5-(4S,5R) peut être préparé de la manière suivante :

A une solution agitée de 12,8 g de tert-butoxycarbonyl-3 diméthyl-2,2 phényl-4 oxazolidinecarboxylate-5-(4S,5R) d'éthyle dans 200 cm3 d'éthanol, on ajoute en 10 minutes une solution de 4,62 g d'hydrate d'hydroxyde de lithium dans 80 cm3 d'eau. Après 10 minutes d'agitation supplémentaire, le milieu réactionnel est concentré à sec sous pression réduite (2,7 kPa) à une température voisine de 40°C. Le résidu obtenu est dissous dans 70 cm3 d'eau puis extrait avec 3 fois 20 cm3 d'oxyde d'isopropyle. La phase aqueuse est ensuite acidifiée à un pH voisin de 2,6 par addition d'environ 100 cm3 d'une solution aqueuse 1N d'acide chlorhydrique, puis extraite 3 fois avec 50 cm3 de dichlorométhane. Les phases organiques réunies sont séchées sur du sulfate de magnésium, filtrées puis concentrées à sec sous pression réduite (2,7 kPa) à une température voisine de 40°C. On obtient ainsi 11,3 g d'acide tert-butoxycarbonyl-3 diméthyl-2,2 phényl-4 oxazolidinecarboxylique-5-(4S,5R) sous forme d'une huile jaune dont les caractéristiques sont les suivantes : - pouvoir rotatoire : [α]²⁰_{D} -3,3° (c = 0,8 ; CHCl₃) - spectre de RMN (250 MHz ; déplacements en ppm)

| | |
|---|---|
| 7,4 ppm m, 5H, C₆H₅ | |
| 5,2 ppm m, 1H, CHN | |
| 4,55 ppm | d, 1H, CHO |
| 1,85 ppm | s, 3H, C-CH₃ |
| 1,75 ppm | s, 3H, C-CH₃ |
| 1,2 ppm m, 9H, C(CH₃)₃ | |

Le tert-butoxycarbonyl-3 diméthyl-2,2 phényl-4 oxazolidinecarboxylate-5-(4S,5R) d'éthyle peut être préparé de la manière suivante :

Une solution de 11,7 g de tert-butoxycarbonylamino-3 hydroxy-2 phényl-3 propionate-(2R,3S) d'éthyle, de 3,6 cm3 de méthoxy-2 propène et de 0,06 g d'acide p.toluènesulfonique dans 120 cm3 de toluène est agitée pendant 1 heure à une température voisine de 20°C. Le milieu réactionnel est porté à ébullition, puis on ajoute 0,06 g d'acide p.toluènesulfonique. Le distillat est recueilli dans un récipient gradué tandis que l'on additionne, goutte à goutte, une solution de 18 cm3 de méthoxy-2 propène dans 82 cm3 de toluène de manière à maintenir constant le volume du milieu réactionnel. Après 1 heure 20 minutes de distillation on ajoute 0,06 g d'acide paratoluènesulfonique, puis la distillation est poursuivie pendant 10 minutes ; le volume de distillat recueilli est alors de 100 cm3. Le milieu réactionnel est refroidi à une température voisine de 20°C, on ajoute alors 25 cm3 d'une solution aqueuse saturée d'hydrogénocarbonate de sodium. La phase aqueuse est décantée, puis extraite avec 2 fois 10 cm3 de dichlorométhane ; les phases organiques réunies sont séchées sur du sulfate de magnésium, filtrées et concentrées à sec sous pression réduite (2,7 kPa) à une température voisine de 40°C. On obtient ainsi 20,8 g d'une huile jaune qui est purifiée par chromatographie sur 630 g de gel de silice (diamètre de la colonne : 5,5 cm, éluant: cyclohexane-acétate d'éthyle [70-30 en volumes], fractions de 100 cm3). Après concentration à sec sous pression réduite (2,7 kPa) à une température voisine de 40°C des fractions 3 à 9 on obtient 13 g de tert-butoxycarbonyl-3 diméthyl-2,2 phényl-4 oxazolidinecarboxylate-5-(4S,5R) d'éthyle sous forme d'une huile jaune dont les caractéristiques sont les suivantes :
- pouvoir rotatoire : [α]²⁰_{D} = -7,3° (c = 1 ; CHCl₃)
- spectre de RMN (250 MHz ; déplacements en ppm) :

| | |
|---|---|
| 7,3 ppm m, 5H, C₆H₅ | |
| 5,05 ppm | m, 1H, CHN |
| 4,45 ppm | d, 1H, CHO |
| 4,25 ppm | q, 2H, OCH₂- |
| 1,8 ppm s, 3H, C-CH₃ | |
| 1,7 ppm s, 3H, C-CH₃ | |
| 1,3 ppm t, 3H, O-C-CH₃ | |
| 1,1 ppm m, 9H, C(CH₃)₃ | |

Le tert-butoxycarbonylamino-3 hydroxy-2 phényl-3 propionate-(2R,3S) d'éthyle peut être préparé selon la méthode suivante :

A une solution agitée de 16 g d'amino-3 hydroxy-2 phényl-3 propionate-(2R,3S) d'éthyle dans 160 cm3 de dichlorométhane, on ajoute 7,1 g d'hydrogénocarbonate de sodium puis on coule en 40 minutes une solution de 22,1 g de dicarbonate de di.tert-butyle dans 40 cm3 de dichlorométhane. Le milieu réactionnel est agité 3,25 heures à une température voisine de 20°C, puis on ajoute 150 cm3 d'eau. La phase organique est décantée, la phase aqueuse est extraite avec 50 cm3 de dichlorométhane, puis les phases organiques réunies sont séchées sur du sulfate de magnésium, filtrées et concentrées à sec sous pression réduite (2,7 kPa) à une température voisine de 30°C. Le résidu obtenu est trituré dans 50 cm3 d'éther isopropylique ; le solide obtenu est filtré, puis séché sous pression réduite (2,7 kPa) à une température voisine de 20°C. On obtient ainsi 11,9 g de tertbutoxycarbonylamino-3 hydroxy-2 phényl-3 propionate-(2R,3S) d'éthyle sous forme d'une poudre blanche fondant à 124°C dont le pouvoir rotatoire est [α]²⁰_{D} = 6,3° (c = 1; CHCl₃).

L'amino-3 hydroxy-2 phényl-3 propionate-(2R,3S) d'éthyle peut être préparé par la méthode décrite par H. Hönig et al, Tetrahedron, 46, p.3841 (1990).

### EXEMPLE 2

En opérant d'une manière analogue à celle décrite à l'exemple 1, mais à partir de 10 g d'acide tert-butoxycarbonyl-3 diméthyl-2,2 phényl-4 oxazolidinecarboxylique-5-(4S,5R) et de 12,6 g d'acétoxy-4 benzoyloxy-2α époxy-5β,20 dihydroxy-1,13α oxo-9 bis-(trichloro-2,2,2 éthoxy)carbonyloxy-7β,10β taxène-11, on obtient, après purification par chromatographie flash (éluant : dichlorométhane-méthanol [98-2 en volumes]) et concentration à sec sous pression réduite (2,7 kPa) à une température voisine de 40°C des fractions 22 à 39 on obtient 14 g de tert-butoxycarbonyl-3 diméthyl-2,2 phényl-4 oxazolidinecarboxylate-5-(4S,5R) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche dont les caractéristiques sont les suivantes :
- pouvoir rotatoire : [α]²⁰_{D} = -37,2° (c = 1 ; méthanol)
- spectre de RMN (400 MHz ; déplacements en ppm)

| | |
|---|---|
| 7,4 à 7,2 ppm | m, 5H, C₆H₅ (chaîne) |
| 6,25 ppm | t, 1H, H-13 |
| 6,2 ppm s, 1H, H-10 | |
| 5,55 ppm | dd, 1H, H-7 |
| 5,1 ppm m, 1H, CHN (H-4') | |
| 4,9 et 4,6 ppm | 2d, 2H, CH₂CCl₃ |
| 7,78 ppm | s, 2H, CH₂CCl₃ |
| 4,45 ppm | d, 1H, CHO (H-5') |
| 4,1 et 4,28 ppm | 2d, 2H, H-20 |
| 1,1 ppm m, 9H, C(CH₃)₃ | |

L'acétoxy-4 benzoyloxy-2α époxy-5,20 dihydroxy-1,13α oxo-9 bis-(trichloro-2,2,2 éthoxy) carbonyloxy-7β,10β taxène-11 peut être préparé selon la méthode décrite dans le brevet européen EP 0 253 738.

### EXEMPLE 3

En opérant d'une manière analogue à celle décrite à l'exemple 1, mais à partir de 14 g de tert-butoxycarbonyl-3 diméthyl-2,2 phényl-4 oxazolidinecarboxylate-5-(45,5R) d'acétoxy-4 benzoyloxy-2α époxy-5β,20 hydroxy-1 1 oxo-9 bis-(trichloro-2,2,2 éthoxy) carbonyloxy-7β,10β taxène-11 yle-13α, on obtient, après purification par chromatographie flash (éluant : dichlorométhane-méthanol [95-5 en volumes]) et concentration à sec sous pression réduite (2,7 kPa) à une température voisine de 40°C des fractions 7 et 8 on obtient 6,3 g d'amino-3 hydroxy-2 phényl-3 propionate-(2R,3S) d'acétoxy-4 benzoyloxy-2α époxy-5β,20 hydroxy-1 oxo-9 bis-(trichloro-2,2,2 éthoxy) éthoxy)carbonyloxy-7β,10β taxène-11 yle-13α sous forme d'une meringue blanche dont les caractéristiques sont les suivantes :
- pouvoir rotatoire : [α]²⁰_{D} = -39,7° (c = 1; méthanol)
- spectre de RMN (400 MHz ; déplacements en ppm)

| | |
|---|---|
| 7,45 à 7,3 ppm | m, 5H, C₆H₅ (chaîne) |
| 6,3 ppm s, 1H, 11-10 | |
| 6,22 ppm | t, 1H, H-13 |
| 5,6 ppm dd, 1H, H-7 | |
| 4,95 et 4,65 ppm | 2d, 2H, CH₂CCl₃ |
| 4,8 ppm s, 2H, CH₂CCl₃ | |
| 4,40 ppm | m, 3H, H-2'+ H-3' + H-20 |
| 4,2 ppm d, 1H, H-20 | |

L'amino-3 hydroxy-2 phényl-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α est transformé en tert-butoxycarbonylamino-3 hydroxy-2 phényl-3 propionate-(2R,3S) d'acétoxy-4 benzoyloxy-2α époxy-5β,20 hydroxy-1 oxo-9 bis- (trichloro-2,2,2 éthoxy)carbonyloxy-7β,10β taxène-11 yle-13α par action du dicarbonate de di.tert-butyle en présence d'une base minérale ou organique.

Le tert-butoxycarbonylamino-3 hydroxy-2 phényl-3 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α est transformé en tert-butoxycarbonylamino-3 hydroxy-2 phényl-3 propionate-(2R,3S) d'acétoxy-4 benzoyloxy-2α époxy-5β,20 trihydroxy-1,7β,10β oxo-9 taxène-11 yle-13α par remplacement des groupements trichloro-2,2,2 éthoxycarbonyle par un atome d'hydrogène au moyen de zinc en présence d'acide acétique selon la méthode décrite dans la demande de brevet européen EP 0 253 738.

### EXEMPLE 4

En opérant d'une manière analogue à celle décrite à l'exemple 1, mais à partir de 0,54 g d'acide tert-butoxycarbonyl-3 diméthyl-2,2 phényl-4 oxazolidinecarboxylique-5-(4SR,5RS) et de 0,47 g d'acétoxy-4 benzoyloxy-2α époxy-5β,10 dihydroxy-1,13α oxo-9 bis-(trichloro-2,2,2 éthoxy) carbonyloxy-7β,10β taxène-11, on obtient, après purification par chromatographie flash (éluant : dichlorométhane-méthanol [99,5-0,5 en volumes]) et concentration à sec des fractions 9 à 11 sous pression réduite (2,7 kPa) à une température voisine de 40°C, 0,5 g de tert-butoxycarbonyl-3 diméthyl-2,2 phényl-4 oxazolidinecarboxylate-5-(4S,5R) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche dont les caractéristiques sont identiques à celles du produit obtenu à l'exemple 2.

L'acide tert-butoxycarbonyl-3 diméthyl-2,2 phényl-4 oxazolidinecarboxylique-5-(4SR,5RS) peut être obtenu dans les conditions décrites dans l'exemple 1 pour la préparation de l'acide tert-butoxycarbonyl-3 diméthyl-2,2 phényl-4 oxazolidinecarboxylique-5- (4S,5R).

Ainsi, à partir de 5 g de tert-butoxycarbonyl-3 diméthyl-2,2 phényl-4 oxazolidinecarboxylate-5-(4SR,5RS) d'éthyle, on obtient 4,54 g d'acide tert-butoxycarbonyl-3 diméthyl-2,2 phényl-4 oxazolidinecarboxylique-5-(4SR,5RS) sous forme d'un solide blanc fondant à 110°C dont les caractéristiques sont les suivantes :
- spectre de RMN (250 MHz ; déplacements en ppm)

| | |
|---|---|
| 7,4 ppm m, 5H, C₆H₅ | |
| 5,2 ppm m, 1H, CHN | |
| 4,55 ppm | d, 1H, CHO |
| 1,85 ppm | s, 3H, C-CH₃ |
| 1,75 ppm | s, 3H, C-CH₃ |
| 1,2 ppm m, 9H, C(CH₃)₃ | |

Le tert-butoxycarbonyl-3 diméthyl-2,2 phényl-4 oxazolidinecarboxylate-5-(4SR,5RS) d'éthyle peut être préparé de la manière suivante :

Une solution de 0,42 g de tert-butoxycarbonylamino-3 hydroxy-2 phényl-3 propionate-(2RS,3SR) d'éthyle, de 1 cm3 de diméthoxy-2,2 propane et de 0,01 g d'acide p.toluènesulfonique dans 10 cm3 de toluène est portée à l'ébullition pendant 15 minutes; on recueille 5 cm3 de distillat. On ajoute alors 5 cm3 de toluène et 1 cm3 de diméthoxy-2,2 propane, puis distille de nouveau 5 cm3 de distillat en 15 minutes. Ce processus est répété 2 fois. Le milieu réactionnel est refroidi à une température voisine de 20°C, on ajoute alors 20 cm3 d'une solution saturée d'hydrogénocarbonate de sodium. La phase aqueuse est décantée, puis extraite avec 20 cm3 de dichlorométhane ; les phases organiques réunies sont séchées sur du sulfate de magnésium, filtrées et concentrées à sec sous pression réduite (2,7 kPa) à une température voisine de 40°C. On obtient ainsi 0,42 g d'une huile jaune qui est purifiée par chromatographie sur 15 g de gel de silice (diamètre de la colonne : 1,5 cm ; éluant : dichlorométhane ; fractions de 10 cm3). Après concentration à sec sous pression réduite (2,7 kPa) à une température voisine de 40°C des fractions 6 à 13 on obtient 0,2 g de tert-butoxycarbonyl-3 diméthyl-2,2 phényl-4 oxazolidinecarboxylate-5-(4SR,5RS) d'éthyle sous forme d'une huile jaune dont les caractéristiques sont les suivantes :
- spectre de RMN (250 MHz ; déplacements en ppm)

| | |
|---|---|
| 7,3 ppm m, 5H, C₆H₅ | |
| 5,05 ppm | m, 1H, CHN |
| 4,45 ppm | d, 1H, CHO |
| 4,25 ppm | q, 2H, OCH₂- |
| 1,8 ppm s, 3H, C-CH₃ | |
| 1,7 ppm s, 3H, C-CH₃ | |
| 1,3 ppm t, 3H, O-C-CH₃ | |
| 1,1 ppm m, 9H, C(CH₃)₃ | |

Le tert-butoxycarbonylamino-3 hydroxy-2 phényl-3 propionate-(2RS,3SR) d'éthyle peut être préparé selon la méthode décrite à l'exemple 1 pour la préparation du tert-butoxycarbonylamino-3 hydroxy-2 phényl-3 propionate-(2R,3S) d'éthyle.

Ainsi, à partir de 0,78 g d'amino-3 hydroxy-2 phényl-3 propionate-(2RS,3SR) d'éthyle, on obtient 0,6 g de tert-butoxycarbonylamino-3 hydroxy-2 phényl-3 propionate-(2RS,3SR) d'éthyle sous forme d'une poudre blanche fondant à 102°C.

L'amino-3 hydroxy-2 phényl-3 propionate-(2RS,3SR) d'éthyle peut être préparé par la méthode décrite par H. Hönig et al., Tetrahedron, 46, p.3841 (1990).

### EXEMPLE 5

En opérant d'une manière analogue à celle décrite à l'exemple 1, mais à partir de 1,7 g d'acide tert-butoxycarbonyl-3 diéthyl-2,2 phényl-4 oxazolidinecarboxylique-5-(4S,5R) et de 2,9 g d'acétoxy-4 benzoyloxy-2α époxy-5β,20 dihydroxy-1,13α oxo-9 bis-(trichloro-2,2,2 éthoxy) carbonyloxy-7β,10β taxène-11, on obtient 3,5 g de tert-butoxycarbonyl-3 diéthyl-2,2 phényl-4 oxazolidinecarboxylate-5-(4S,5R) d'acétoxy4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.
- pouvoir rotatoire : [α]²⁰_{D} = -41,2° (c = 1 ; méthanol).

Le tert-butoxycarbonyl-3 diéthyl-2,2 phényl-4 oxazolidinecarboxylate-5-(4S,5R) d'acétoxy-4 benzoyloxy2α époxy-5β,20 hydroxy-1 oxo-9 bis-(trichloro-2,2,2 éthoxy) carbonyloxy-7β,10β taxène-11 yle-13α est transformé en amino-3 hydroxy-2 phényl-3 propionate-(2R,3S) d'acétoxy-4 benzoyloxy2a époxy-5β,20 hydroxy-1 oxo-9 bis-(trichloro-2,2,2 éthoxy) carbonyloxy-7β,10β taxène-11 yle-13α par action de l'acide formique comme décrit à l'exemple 1 pour le tert-butoxycarbonyl-3 diméthyl-2,2 phényl-4 oxazolidinecarboxylate-5-(4S,5R) d'acétoxy-4 benzoyloxy2a époxy-5β,20 hydroxy-1 oxo-9 bis-(trichloro-2,2,2 éthoxy) carbonyloxy-7β,10β taxène-11 yle-13α. Le produit ainsi obtenu possède des caractéristiques physiques en tous points identiques à celles décrites à l'exemple 4 pour le même produit.
- pouvoir rotatoire : [α]²⁰_{D} = -38,3° (c = 0,8 ; méthanol).

L'acide tert-butoxycarbonyl-3 diéthyl-2,2 phényl-4 oxazolidinecarboxylique-5-(4S,5R) peut être obtenu dans les conditions décrites à l'exemple 1 pour la préparation de l'acide tert-butoxycarbonyl-3 diméthyl-2,2 phényl-4 oxazolidinecarboxylique-5-(4S,5R). Ainsi à partir de 2,3 g de tert-butoxycarbonyl-3 diéthyl-2,2 phényl-4 oxazolidinecarboxylate-5-(4S,5R) d'éthyle, on obtient 1,7 g d'acide tert-butoxycarbonyl-3 diéthyl-2,2 phényl-4 oxazolidinecarboxylique-5-(4S,5R) sous forme de cristaux blancs fondant à 185°C.
- pouvoir rotatoire : [α]²⁰_{D} = +2,4° (c = 0,5 ; méthanol).

Le tert-butoxycarbonyl-3 diéthyl-2,2 phényl-4 oxazolidinecarboxylate-5-(4S,5R) d'éthyle peut être préparé de la manière suivante :

Une solution de 2,5 g de tert-butoxycarbonylamino-3 hydroxy-2 phényl-3 propionate-(2R,3S) d'éthyle, de 1,12 g de diméthoxy-3,3 pentane et de 25 mg de p-toluènesulfonate de pyridinium dans 25 cm3 de toluène est agitée pendant 3 heures à une température voisine de 20°C. Le mélange réactionnel est chauffé jusqu'à l'ébullition et le distillat est recueilli dans un récipient gradué. Après avoir recueilli 20 cm3 de distillat on additionne une solution de 1,12 g de diméthoxy-3,3 pentane et de 25 mg de p-toluènesulfonate de pyridinium dans 10 cm3 de toluène au milieu réactionnel et chauffe au reflux du toluène pendant 5 heures. Le milieu réactionnel est refroidi à une température voisine de 20°C, on ajoute ensuite 10 cm3 d'une solution aqueuse saturée d'hydrogénocarbonate de sodium. La phase aqueuse est décantée, puis extraite par 2 fois 10 cm3 de dichlorométhane. Les phases organiques sont réunies, séchées sur sulfate de magnésium, filtrées et concentrées à sec sous pression réduite (2,7 kPa) à une température voisine de 40°C. On obtient ainsi 2,3 g de tert-butoxycarbonyl-3 diéthyl-2,2 phényl-4 oxazolidinecarboxylate-5-(4S,5R) d'éthyle sous forme d'une huile jaune.
- pouvoir rotatoire : [α]²⁰_{D} = -8,9° (c = 1 ; méthanol).

Le diméthoxy-3,3 pentane peut être préparé par la méthode décrite par Lorette et al., J. Org. Chem., 24, 1731, (1959).

### EXEMPLE 6

En opérant d'une manière analogue à celle décrite à l'exemple 1, mais à partir de 0,9 g d'acide tert-butoxycarbonyl-3 cyclohexane-spiro-2 phényl-4 oxazolidinecarboxylique-5-(4S,5R) et de 1,443 g d'acétoxy-4 benzoyloxy2α époxy-5β,20 dihydroxy-1,13α oxo-9 bis-(trichloro-2,2,2 éthoxy) carbonyloxy-7β,10β taxène-11, on obtient 1,85 g de tert-butoxycarbonyl-3 cyclohexane-spiro-2 phényl-4 oxaolidinecarboxylate-5-(4S,5R) d'acétoxy-4 benzoyloxy2α époxy-5β,20 hydroxy-1 oxo-9 bis-(trichloro-2,2,2 éthoxy) carbonyloxy-7β,10β taxène-11 yle-13α sous forme d'une meringue blanche.

Le tert-butoxycarbonyl-3 cyclohexane-spiro-2 phényl-4 oxazolidinecarboxylate-5-(4S,5R) d'acétoxy-4 benzoyloxy2α époxy-5β,20 hydroxy-1 oxo-9 bis-(trichloro-2,2,2 éthoxy) carbonyloxy-7β,10β taxène-11 yle-13α est transformé en amino-3 hydroxy-2 phényl-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α par action de l'acide formique comme décrit à l'exemple 1 pour le tert-butoxycarbonyl-3 diméthyl-2,2 phényl-4 oxazolidinecarboxylate-5-(4S,5R) d'acétoxy-4 benzoyloxy-2α époxy-5β,20 hydroxy-1 oxo-9 bis-(trichloro-2,2,2 éthoxy)carbonyloxy-7β,10β taxène-11 yle-13α. Le produit ainsi obtenu possède des caractéristiques physiques en tous points identiques à celles décrites à l'exemple 3 pour le même produit.
- pouvoir rotatoire : [α]²⁰_{D} = -41,7° (c = 0,5 ; méthanol).

L'acide tert-butoxycarbonyl-3 cyclohexane-spiro-2 phényl-4 oxazolidinecarboxylique-5-(4S,5R) peut être obtenu dans les conditions décrites à l'exemple 1 pour la préparation de l'acide tert-butoxycarbonyl-3 diméthyl-2,2 phényl-4 oxazolidinecarboxylique-5-(4S,5R). Ainsi à partir de 1,2 g de tert-butoxycarbonyl-3 cyclohexane-spiro-2 phényl-4 oxazolidinecarboxylate-5-(4S,5R) d'éthyle, on obtient 1 g d'acide tert-butoxycarbonyl-3 cyclohexane-spiro-2 phényl-4 oxazolidinecarboxylique-5-(4S,5R) sous forme d'une meringue blanche.
- pouvoir rotatoire : [α]²⁰_{D} = -4,5° (c = 0,5 ; méthanol).

Le tert-butoxycarbonyl-3 cyclohexane-spiro-2 phényl-4 oxazolidinecarboxylate-5-(4S,5R) d'éthyle peut être préparé de la manière suivante :

Une solution de 2,5 g de tert-butoxycarbonylamino-3 hydroxy-2 phényl-3 propionate-(2R,3S) d'éthyle, de 1,22 g de diméthoxy-1,1 cyclohexane et de 25 mg de p-toluènesulfonate de pyridinium dans 25 cm3 de toluène est agitée pendant 3 heures à une température voisine de 20°C. Le mélange réactionnel est chauffé jusqu'à l'ébullition et le distillat est recueilli dans un récipient gradué. Après avoir recueilli 20 cm3 de distillat on additionne une solution de 1,22 g de diméthoxy-1,1 cyclohexane et de 25 mg de p-toluènesulfonate de pyridinium dans 10 cm3 de toluène au milieu réactionnel et chauffe au reflux du toluène pendant 30 heures. Le milieu réactionnel est refroidi à une température voisine de 20°C, on ajoute ensuite 10 cm3 d'une solution aqueuse saturée d'hydrogénocarbonate de sodium. La phase aqueuse est décantée, puis extraite par 2 fois 10 cm3 de dichlorométhane. Les phases organiques sont réunies, séchées sur du sulfate de magnésium, filtrées et concentrées à sec sous pression réduite (2,7 kPa) à une température voisine de 40°C. On obtient 2,6 g d'une meringue blanche que l'on purifie par chromatographie sur 100 g de silice (0,063-0,2 mm) contenus dans une colonne de 2 cm de diamètre (éluant : dichlorométhane) en recueillant des fractions de 25 cm3. Les fractions 6 à 13 sont réunies et concentrées à sec sous pression réduite (0,27 kPa) à une température voisine de 40°C. On obtient ainsi 1,25 g de tert-butoxycarbonyl-3 cyclohexanespiro-2 phényl-4 oxazolidinecarboxylate-5-(4S,5R) d'éthyle sous forme d'une huile jaune.

Le diméthoxy-1,1 cyclohexane peut être préparé par la méthode décrite par Lorette et al., J. Org. Chem., 24, 1731, (1959).

## Revendications

1. Procédé de préparation de dérivés du taxane de formule générale : 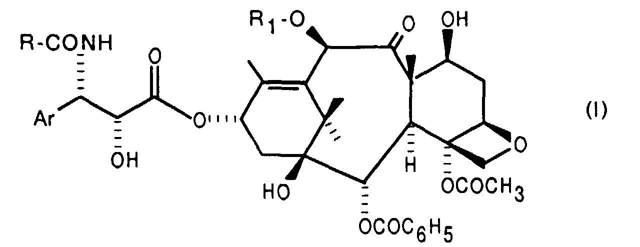 dans laquelle R représente un radical t.butoxy ou phényle, R₁ représente un atome d'hydrogène ou un radical acétyle et Ar représente un radical phényle, caractérisé en ce que :
a) on condense un dérivé de l'oxazolidine de formule générale : 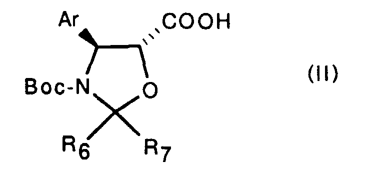 dans laquelle Ar est défini comme précédemment, Boc représente le radical t.butoxycarbonyle et R₆ et R₇, identiques ou différents, représentent un radical alkyle contenant 1 à 4 atomes de carbone éventuellement substitué par un ou plusieurs radicaux aryles (phényle), ou aryle (phényle), ou bien R₆ et R₇ forment ensemble avec l'atome de carbone auquel ils sont liés un cycle ayant de 4 à 7 chaînons, sur un dérivé du taxane de formule générale : 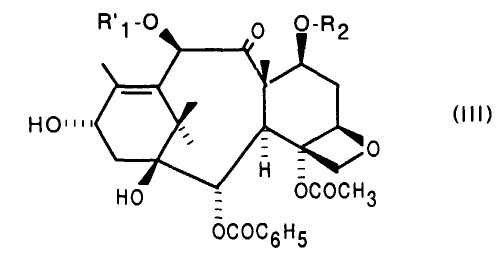 dans laquelle R'₁ représente un radical acétyle ou un groupement protecteur de la fonction hydroxy et R₂ représente un groupement protecteur de la fonction hydroxy, pour obtenir un produit de formule générale : 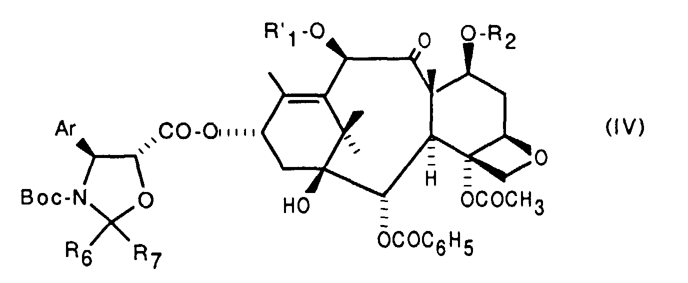 dans laquelle Ar, R'₁, R₂, R₆ et R₇ sont définis comme précédemment,
b) on traite en milieu acide, dans des conditions qui sont sans effet sur R'₁ et R₂, pour obtenir le produit de formule générale : 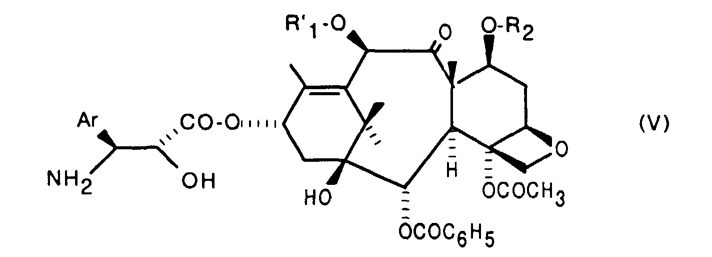 dans laquelle R'₁ et R₂ sont définis comme précédemment,
c) on traite par un réactif convenable permettant d'introduire un radical t.butoxycarbonyle ou benzoyle sur la fonction amino, pour obtenir un produit de formule générale : 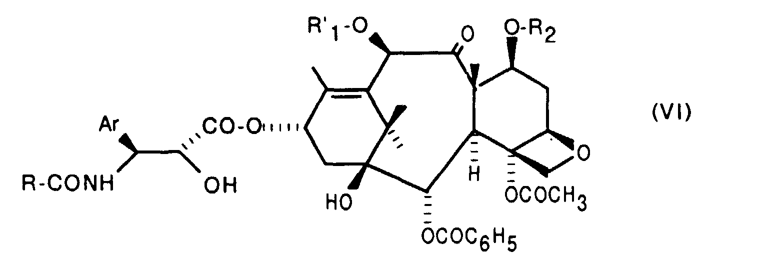
d) on remplace les groupements protecteurs R'₁ et R₂ par des atomes d'hydrogène dans des conditions appropriées puis,
e) on isole le produit obtenu et éventuellement le purifie.

2. Procédé selon la revendication 1 caractérisé en ce que les radicaux protecteurs représentés par R'₁ et R₂ sont des radicaux trichloro-2,2,2 éthoxycarbonyle ou des radicaux pouvant être transformés en radicaux trichloro-2,2,2 éthoxycarbonyle.

3. Procédé selon l'une des revendications 1 ou 2 caractérisé en ce que la condensation du produit de formule générale (II) sur le dérivé du taxane de formule générale (III) est effectuée en présence d'un agent de condensation et d'un agent d'activation.

4. Procédé selon l'une des revendications 1 à 3 caractérisé en ce que le traitement sélectif du produit de formule générale (IV) est effectué au moyen d'un acide minéral ou organique éventuellement dans un alcool.

5. Procédé selon l'une des revendications 1 à 4 caractérisé en ce que l'introduction d'un radical t.butoxycarbonyle ou benzoyle sur le produit de formule générale (V) est effectuée au moyen de dicarbonate de di-t.butyle ou de chlorure de benzoyle.

6. Procédé selon l'une des revendications 1 à 5 caractérisé en ce que le remplacement des groupements protecteurs représentés par R'₁ et R₂ par des atomes d'hydrogène est effectué par le zinc en milieu acide acétique ou au moyen d'un acide minéral ou organique dans un alcool aliphatique en présence de zinc.

7. Les dérivés de l'oxazolidine de formule générale : 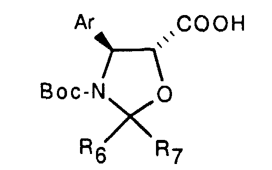 dans laquelle Ar est défini selon l'une des revendications 1 ou 2, Boc représente le radical t.butoxycarbonyle et R₆ et R₇, identiques ou différents, représentent un radical alkyle contenant 1 à 4 atomes de carbone éventuellement substitué par un ou plusieurs radicaux aryles (phényle), ou aryle (phényle), ou bien R₆ et R₇ forment ensemble avec l'atome de carbone auquel ils sont liés un cycle ayant de 4 à 7 chaînons.

8. Procédé de préparation d'un dérivé du l'oxazolidine selon la revendication 7 caractérisé en ce que l'on fait réagir un méthoxyalcène éventuellement substitué par un ou plusieurs radicaux aryles, un diméthoxyalcane éventuellement substitué par un ou plusieurs radicaux aryles ou un gem-diméthoxycycloalcane contenant 4 à 7 atomes de carbone sur un dérivé de la phénylisosérine de formule générale : 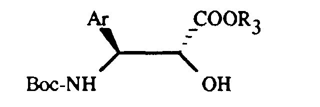 dans laquelle Ar est défini selon la revendication 1, Boc représente le radical t.butoxycarbonyle et R₃ représente un radical alkyle contenant 1 à 4 atomes de carbone éventuellement substitué par un radical phényle, puis saponifie le produit obtenu.

## Patentansprüche

1. Verfahren zur Herstellung von Taxan-Derivaten der allgemeinen Formel (I) 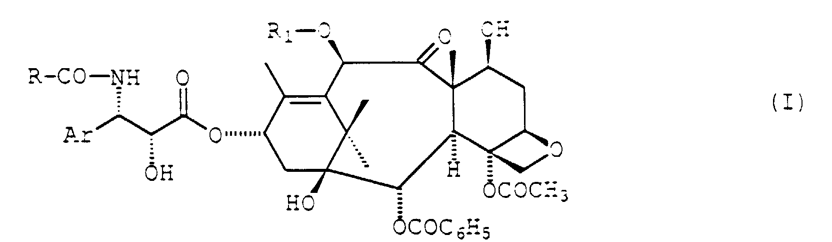 in der R einen Rest tert.-Butoxy oder Phenyl darstellt, R₁ ein Wasserstoffatom oder ein Rest Acetyl ist und Ar einen Rest Phenyl bedeutet, dadurch gekennzeichnet, daß man
a) ein Derivat von Oxazolidin der allgemeinen Formel (II) 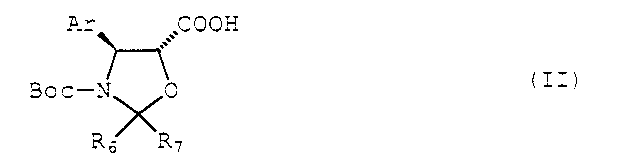 in der Ar wie oben definiert ist, Boc den Rest tert.-Butoxycarbo-nyl darstellt und R₆ und R₇, gleich oder verschieden, einen Rest Alkyl mit 1 bis 4 Kohlenstoffatomen bedeuten, gegebenenfalls substituiert durch einen Rest Aryl (Phenyl) oder mehrere Reste Aryl (Phenyl), oder R₆ und R₇, zusammen mit dem Kohlenstoffatom, an das sie gebunden sind, einen Ring mit 4 bis 7 Ringgliedern bilden, mit einem Taxan-Derivat der allgemeinen Formel (III) 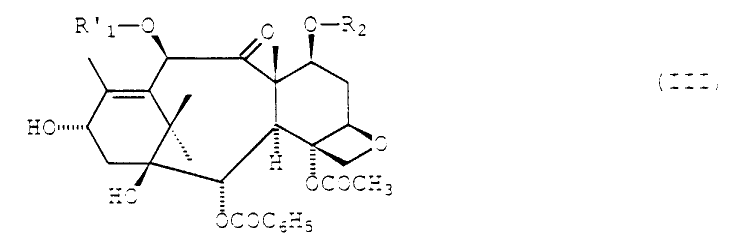 kondensiert, in der R'₁ einen Rest Acecyl oder eine Schutzgruppe für die Hydroxyfunktion darstellt und R₂ eine Schutzgruppe für die Hydroxyfunktion ist, um ein Produkt der allgemeinen Formel (IV) 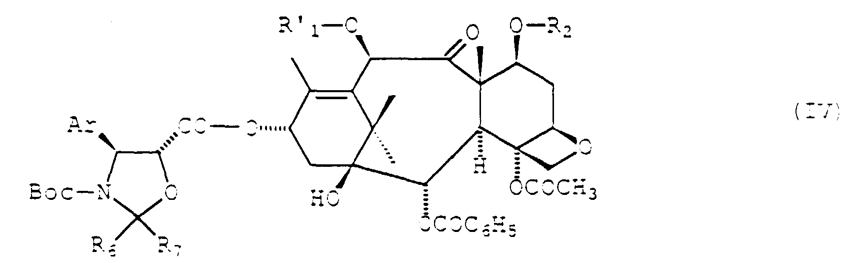 zu erhalten, in der Ar, R'₁, R₂, R₆ und R₇, wie oben definiert sind,
b) im sauren Medium unter Bedingungen behandelt, die ohne Einfluß auf R'₁ und R₂ sind, um das Produkt der allgemeinen Formel (V) 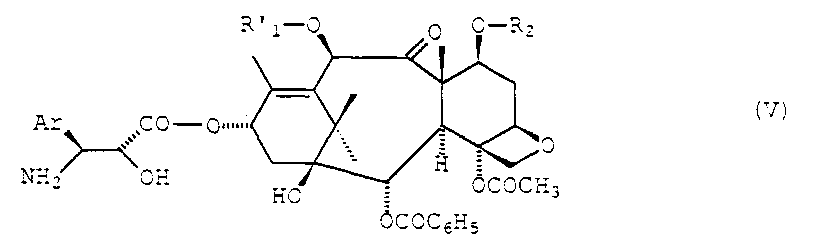 zu erhalten, in der R'₁ und R₂, wie oben definiert sind,
c) mit einem geeigneten Reaktanden behandelt, der es ermöglicht, einen Rest tert.-Butoxycarbonyl oder Benzoyl bei der Aminfunktion einzuführen, um ein Produkt der allgemeinen Formel (VI) 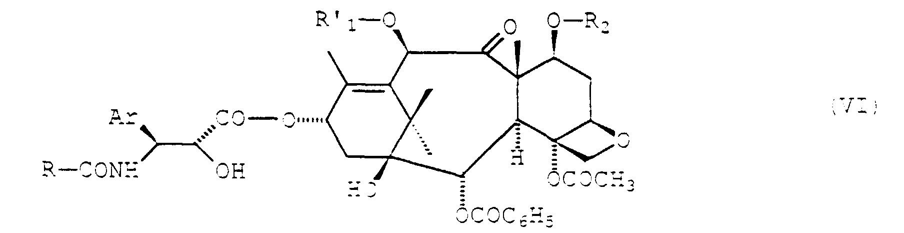 zu erhalten,
d) die Schutzgruppen R'₁ und R₂ unter geeigneten Bedingungen durch Wasserstoffatome austauscht, und anschließend
e) das erhaltene Produkt isoliert und gegebenenfalls reinigt.

2. Verfahren nach Anspruch 1, dadurch gekennzeichnet, daß die durch R'₁ und R₂ dargestellten schützenden Reste die Reste 2,2,2-Trichlor-ethoxycarbonyl oder Reste sind, die in Reste 2,2,2-Trichlor-ethoxycarbonyl umgewandelt werden können.

3. Verfahren nach einem der Ansprüche 1 oder 2, dadurch gekennzeichnet, daß die Kondensation des Produktes der allgemeinen Formel (II) mit dem Taxan-Derivat der allgemeinen Formel (III) in Anwesenheit eines Kondensationsmittels und eines Aktivierungsmittels durchgeführt wird.

4. Verfahren nach einem der Ansprüche 1 bis 3, dadurch gekennzeichnet, daß die selektive Behandlung des Produktes der allgemeinen Formel (IV) mit Hilfe einer Mineralsäure oder organischen Säure, gegebenenfalls in einem Alkohol durchgeführt wird.

5. Verfahren nach einem der Ansprüche 1 bis 4, dadurch gekennzeichnet, daß die Einführung eines Restes tert.-Butoxycarbonyl oder Benzoyl bei dem Produkt der allgemeinen Formel (V) mit Hilfe von Di-tert.-Butyldicarbonat oder Benzoylchlorid durchgeführt wird.

6. Verfahren nach einem der Ansprüche 1 bis 5, dadurch gekennzeichnet, daß der Austausch der durch R'₁ und R₂ dargestellten Schutzgruppen durch Wasserstoffatome mit Hilfe von Zink im Medium von Essigsäure oder auch mit Hilfe einer Mineralsäure oder organischen Säure in einem aliphatischen Alkohol in Anwesenheit von Zink durchgeführt wird.

7. Oxazolidin-Derivate der allgemeinen Formel 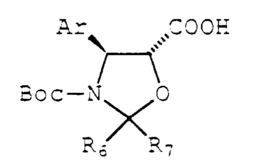 in der Ar wie in einem der Ansprüche 1 oder 2 definiert ist, Boc den Rest tert.-Butoxycarbonyl darstellt und R₆ und R₇, gleich oder verschieden, einen Rest Alkyl mit 1 bis 4 Kohlenstoffatomen bedeuten, gegebenenfalls substituiert durch einen Rest Aryl (Phenyl) oder mehrere Reste Aryl (Phenyl), oder R₆ und R₇ zusammen mit dem Kohlenstoffatom, an das sie gebunden sind, einen Ring mit 4 bis 7 Ringgliedern bilden.

8. Verfahren zur Herstellung eines Oxazolidin-Derivates nach Anspruch 7, dadurch gekennzeichnet, daß man ein Methoxyalken, gegebenenfalls substituiert durch einen oder mehrere Reste Aryl, ein Dimethoxyalkan, gegebenenfalls substituiert durch einen cder mehrere Reste Aryl oder ein gem.-Dimethoxycycloalkan mit 4 bis 7 Kohlenstoffatomen mit einem Derivat von Phenylisoserin der allgemeinen Formel 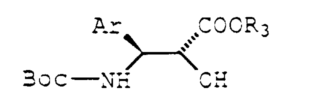 zu Reaktion bringt, in der Ar wie in Anspruch 1 definiert ist, Boc den Rest tert.-Butoxycarbonyl darstellt und R₃ einen Rest Alkyl mit 1 bis 4 Kohlenstoffatomen bedeutet, gegebenenfalls substituiert durch einen Rest Phenyl, und anschließend das erhaltene Produkt verseift.

## Claims

1. Process for preparing taxane derivatives of general formula: 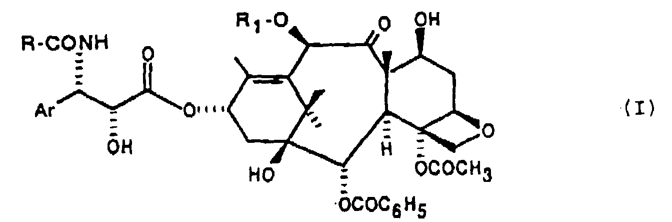 in which R represents a t-butoxy or phenyl radical, R₁ represents a hydrogen atom or an acetyl radical and Ar represents a phenyl radical, characterized in that:
a) an oxazolidine derivative of general formula: 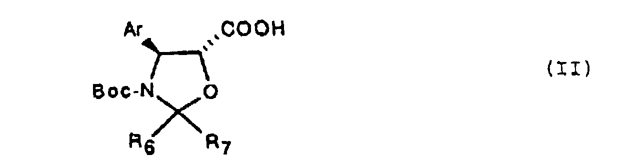 in which Ar is defined as above, Boc represents a t-butoxycarbonyl radical and R₆ and R₇, which may be identical or different, represent an alkyl radical containing 1 to 4 carbon atoms, optionally substituted with one or more aryl (phenyl) radicals, or an aryl (phenyl), or alternatively R₆ and R₇, together with the carbon atom to which they are linked, form a 4- to 7-membered ring, is condensed with a taxane derivative of general formula: 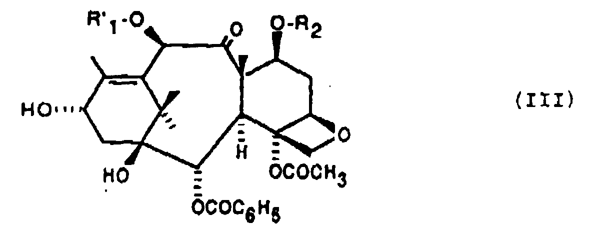 in which R'₁ represents an acetyl radical or a group protecting the hydroxyl function and R₂ represents a group protecting the hydroxyl function, to obtain a product of general formula: 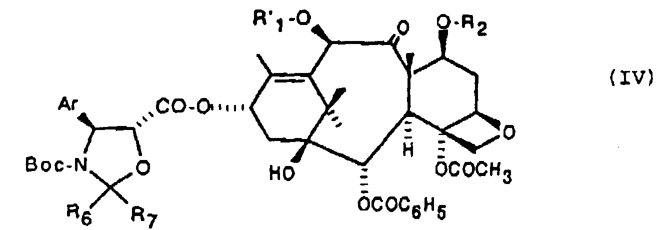 in which Ar, R'₁, R₂, R₆ and R₇ are defined as above,
b) the product is treated in an acid medium, under conditions which have no effect on R'₁ and R₂, to obtain the product of general formula: 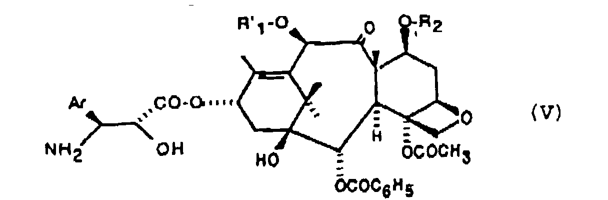 in which R'₁ and R₂ are defined as above,
c) the product is treated with a suitable reagent enabling a t-butoxycarbonyl or benzoyl radical to be introduced on the amino function to obtain a product of general formula: 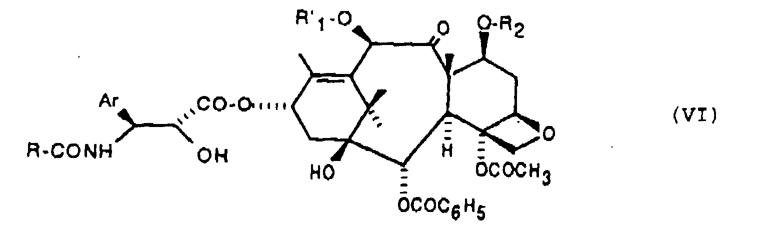
d) the protective groups R'₁ and R₂ are replaced by hydrogen atoms under suitable conditions, and then
e) the product obtained is isolated and, where appropriate, purified.

2. Process according to Claim 1, character-ized in that the protective radicals represented by R'₁ and R₂ are 2,2,2-trichloroethoxycarbonyl radicals or radicals which can be converted to 2,2,2-trichloroethoxycarbonyl radicals.

3. Process according to either of claims 1 and 2, characterized in that the condensation of the product of general formula (II) with the taxane derivative of general formula (III) is performed in the presence of a condensing agent and an activating agent.

4. Process according to one of claims 1 to 3, characterized in that the selective treatment of the product of general formula (IV) is performed by means of an inorganic or organic acid, where appropriate in an alcohol.

5. Process according to one of claims 1 to 4, characterized in that the introduction of a t-butoxycarbonyl or benzoyl radical into the product of general formula (V) is performed by means of di-t-butyl dicarbonate or benzoyl chloride.

6. Process according to one of claims 1 to 5, characterized in that the replacement of the protective groups represented by R'₁ and R₂ by hydrogen atoms is performed with zinc in an acetic acid medium or by means of an inorganic or organic acid in an aliphatic alcohol in the presence of zinc.

7. The oxazolidine derivatives of general formula: 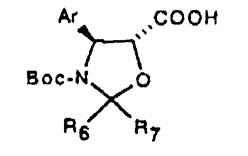 in which Ar is defined according to either of claims 1 and 2, Boc represents a t-butoxycarbonyl radical and R₆ and R₇, which may be identical or different, represent an alkyl radical containing 1 to 4 carbon atoms, optionally substituted with one or more aryl (phenyl) radicals, or an aryl (phenyl), or alternatively R₆ and R₇, together with the carbon atom to which they are linked, form a 4-to 7-membered ring.

8. Process for preparing an oxazolidine derivative according to claim 7, characterized in that a methoxyalkene optionally substituted with one or more aryl radicals, a dimethoxyalkane optionally substituted with one or more aryl radicals or a gem-dimethoxycycloalkane containing 4 to 7 carbon atoms is reacted with a phenylisoserine derivative of general formula: 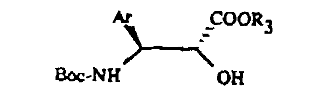 in which Ar is defined according to Claim 1, Boc represents a t-butoxycarbonyl radical and R₃ represents an alkyl radical containing 1 to 4 carbon atoms optionally substituted with a phenyl radical, and the product obtained is then saponified.
